# EUROPEAN PATENT APPLICATION

(11) **EP 4 767 924 A1**
(43) Date of publication of application: **01.07.2026**
(21) Application number: 25196986.1
(22) Date of filing: 20.08.2025
(51) Int. Cl.: A61B 5/00, A61B 5/024, A61B 5/316, A61B 5/349, A61N 1/36

(54) **METHOD AND DEVICE FOR PROCESSING A PHYSIOLOGICAL SIGNAL AND ELECTRICAL STIMULATION THERAPY SYSTEM USING THE SAME**

(30) Priority: 30.12.2024 US 202419005295
(71) Applicant: Industrial Technology Research Institute, 310401 Hsinchu (TW)
(72) Inventor: HU, Chang-Lin, 804 Kaohsiung City (TW); WANG, Yi-Chun, 302 Zhubei City, Hsinchu County (TW); LI, Chien-Ju, 302 Zhubei City, Hsinchu County (TW)
(74) Representative: Krauns, Christian

(57) **Abstract**

A method for processing a physiological signal is provided. The method includes the following steps. A standard physiological signal as a first template to perform a first match filtering on an original physiological signal of a subject under test is used, thereby obtaining a first filtered physiological signal. A correlation evaluation on the first filtered physiological signal is performed and based on an evaluation result, whether interference has been sufficiently eliminated is determined. When determining that the interference has not been sufficiently eliminated, a personal physiological signal of the subject under test is used as a second template to perform a second match filtering on the first filtered physiological signal, thereby obtaining a second filtered physiological signal. A physiological parameter of the subject under test based on the second filtered physiological signal is generated.

## Description

### TECHNICAL FIELD

The technical field relates to a method and a device for processing a physiological signal, and an electrical stimulation therapy system using the same.

### BACKGROUND

When measuring physiological signals (e.g., electrocardiogram (ECG) signals), external interference or interference from electronic devices often causes the physiological signals to contain a significant amount of noise, making it difficult to generate physiological parameters (e.g., heart rate variability (HRV) parameters) from the physiological signals. Therefore, eliminating noise from physiological signals to facilitate the subsequent acquisition of physiological parameters is one of the key areas of focus in the industry.

### SUMMARY

According to one embodiment, a method for processing a physiological signal is provided. The method includes the following steps. A standard physiological signal as a first template to perform a first match filtering on an original physiological signal of a subject under test is used, thereby obtaining a first filtered physiological signal. A correlation evaluation on the first filtered physiological signal is performed and based on an evaluation result, whether interference has been sufficiently eliminated is determined. When determining that the interference has not been sufficiently eliminated, a personal physiological signal of the subject under test is used as a second template to perform a second match filtering on the first filtered physiological signal, thereby obtaining a second filtered physiological signal. A physiological parameter of the subject under test based on the second filtered physiological signal is generated.

According to another embodiment, a physiological signal processing device is provided. The physiological signal processing device includes a first filtering module, a correlation evaluation module, a second filtering module, and a physiological parameter generation module. The first filtering module is configured to use a standard physiological signal as a first template to perform a first match filtering on an original physiological signal of a subject under test, thereby obtaining a first filtered physiological signal. The correlation evaluation module is configured to perform a correlation evaluation on the first filtered physiological signal and determine, based on an evaluation result, whether interference has been sufficiently eliminated. The second filtering module is configured to, when determining that the interference has not been sufficiently eliminated, use a personal physiological signal of the subject under test as a second template to perform a second match filtering on the first filtered physiological signal, thereby obtaining a second filtered physiological signal. The physiological parameter generation module is configured to generate a physiological parameter of the subject under test based on the second filtered physiological signal.

According to an alternative embodiment, an electrical stimulation therapy system is provided. The electrical stimulation therapy system includes an electrical stimulation signal providing module and a physiological signal processing device. The electrical stimulation signal providing module is configured to provide an electrical stimulation signal to a subject under test. The physiological signal processing device includes a first filtering module, a correlation evaluation module, a second filtering module, and a physiological parameter generation module. The first filtering module is configured to use a standard physiological signal as a first template to perform a first match filtering on an original physiological signal of the subject under test, thereby obtaining a first filtered physiological signal. The correlation evaluation module is configured to perform a correlation evaluation on the first filtered physiological signal and determine, based on an evaluation result, whether interference has been sufficiently eliminated. The second filtering module is configured to, when determining that the interference has not been sufficiently eliminated, use a personal physiological signal of the subject under test as a second template to perform a second match filtering on the first filtered physiological signal, thereby obtaining a second filtered physiological signal. The physiological parameter generation module is configured to generate a physiological parameter of the subject under test based on the second filtered physiological signal. The electrical stimulation signal providing module is further configured to adjust or terminate the electrical stimulation signal based on the physiological parameter.

In order to provide a better understanding of the above and other aspects of the present disclosure, embodiments are set forth below and described in detail with reference to the accompanying drawings.

### BRIEF DESCRIPTION OF THE DRAWINGS

FIG. 1 illustrates a flowchart of a physiological signal processing method according to an embodiment of the present disclosure.
FIG. 2 illustrates a flowchart the physiological signal processing method according to another embodiment of the present disclosure.
FIG. 3 illustrates a block diagram of a physiological signal processing device according to an embodiment of the present disclosure.
FIG. 4A illustrates an example of a standard electrocardiogram signal.
FIG. 4B illustrates an example of an electrocardiogram signal measured from a subject under test when no electrical stimulation signal is provided to the subject under test.
FIG. 4C illustrates an example of an electrocardiogram signal obtained by performing match filtering on the measured electrocardiogram signal of FIG. 4B using a match filter with the standard electrocardiogram signal of FIG. 4A as a template.
FIG. 5A illustrates an example of a personal electrocardiogram signal.
FIG. 5B illustrates an example of an electrocardiogram signal measured from a subject under test when no electrical stimulation signal is provided to the subject under test.
FIG. 5C illustrates an example of an electrocardiogram signal obtained by performing match filtering on the measured electrocardiogram signal of FIG. 5B by using a match filter with the personal electrocardiogram signal of FIG. 5A as a template.
FIG. 6A illustrates a partial waveform of a measured electrocardiogram signal that corresponds to FIG. 4B and was obtained from a subject under test when no electrical stimulation signal was provided, without match filtering.
FIG. 6B illustrates part of the magnified measured electrocardiogram signal without match filtering shown in FIG. 6A.
FIG. 6C illustrates a schematic diagram of calculating RR interval counts by using the measured electrocardiogram signal without match filtering shown in FIG. 6B.
FIG. 7A illustrates a partial waveform of a measured electrocardiogram signal after match filtering when no electrical stimulation signal is provided to the subject under test, corresponding to FIG. 4C.
FIG. 7B shows part of the magnified measured electrocardiogram signal in FIG. 7A after match filtering.
FIG. 7C illustrates a schematic diagram of RR interval counting by using the measured electrocardiogram signal after match filtering shown in FIG. 7B.
FIG. 8A illustrates an example of a standard electrocardiogram signal.
FIG. 8B shows an example of a measured electrocardiogram signal when no electrical stimulation signal is provided to the subject under test.
FIG. 8C illustrates an example of a measured electrocardiogram signal with significant noise when an electrical stimulation signal is applied to the subject under test.
FIG. 8D illustrates an example of an electrocardiogram signal obtained by performing match filtering on the electrocardiogram signal with significant noise of FIG. 8C by using a match filter with the standard electrocardiogram signal of FIG. 8A as a template.
FIG. 9A illustrates an example of a personal electrocardiogram signal.
FIG. 9B shows an example of a measured electrocardiogram signal when no electrical stimulation signal is provided to the subject under test.
FIG. 9C illustrates an example of a measured electrocardiogram signal with significant noise when an electrical stimulation signal is applied to the subject under test.
FIG. 9D illustrates an example of an electrocardiogram signal obtained by performing match filtering on the electrocardiogram signal with significant noise of FIG. 9C by using a match filter with the personal electrocardiogram signal of FIG. 9A as a template.
FIG. 10A illustrates a partial waveform of a measured electrocardiogram signal after match filtering which is obtained when an electrical stimulation signal is provided to the subject under test, corresponding to FIG. 8D.
FIG. 10B shows part of the magnified measured electrocardiogram signal after match filtering in FIG. 10A.
FIG. 10C illustrates a schematic diagram of RR interval counting by using the measured electrocardiogram signal shown in FIG. 10B after match filtering with the standard electrocardiogram signal as a template.
FIG. 11A illustrates a partial waveform of the measured electrocardiogram signal obtained when an electrical stimulation signal is provided to the subject under test after match filtering with the personal electrocardiogram signal as a template, corresponding to FIG. 9D.
FIG. 11B shows part of the magnified measured electrocardiogram signal after match filtering in FIG. 11A.
FIG. 11C illustrates a schematic diagram of RR interval counting by using the match-filtered measured electrocardiogram signal after match filtering with the personal electrocardiogram signal as a template shown in FIG. 11B.
FIG. 12 illustrates a block diagram of an electrical stimulation therapy system according to an embodiment of the present disclosure.
FIG. 13 illustrates a flowchart of an electrical stimulation therapy method according to an embodiment of the present disclosure.
FIG. 14 illustrates an application example of the electrical stimulation therapy system shown in FIG. 12 and the electrical stimulation therapy method shown in FIG. 13.
FIG. 15A illustrates an example of an electrocardiogram (ECG) signal as a physiological signal according to an embodiment of the present disclosure.
FIG. 15B illustrates an example of a standard photoplethysmography (PPG) signal as a physiological signal according to an embodiment of the present disclosure.
FIG. 15C illustrates an example of an electroencephalogram (EEG) signal as a physiological signal according to an embodiment of the present disclosure.

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

### DETAILED DESCRIPTION

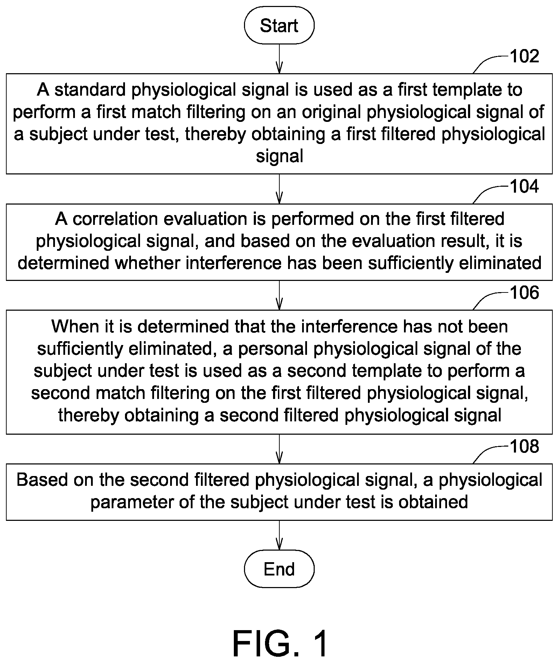

Please refer to FIG. 1, which illustrates a flowchart of a physiological signal processing method according to an embodiment of the present disclosure. The physiological signal processing method of the present disclosure includes the following steps. First, in step 102, a standard physiological signal is used as a first template to perform a first match filtering on an original physiological signal of a subject under test, thereby obtaining a first filtered physiological signal. Next, in step 104, a correlation evaluation is performed on the first filtered physiological signal, and based on the evaluation result, it is determined whether interference has been sufficiently eliminated. Subsequently, in step 106, when it is determined that the interference has not been sufficiently eliminated, a personal physiological signal of the subject under test is used as a second template to perform a second match filtering on the first filtered physiological signal, thereby obtaining a second filtered physiological signal. Thereafter, in step 108, based on the second filtered physiological signal, a physiological parameter of the subject under test is generated.

By performing match filtering twice, interference signals can be effectively removed to ensure the accuracy and stability of physiological signals. This enhances the detection precision of physiological signals, achieves noise suppression, and provides the advantages of dynamic adaptability and diagnostic accuracy. Additionally, it reduces the rate of misjudgment, adapts to various application scenarios, and improves the overall signal processing performance. Further details are provided below.

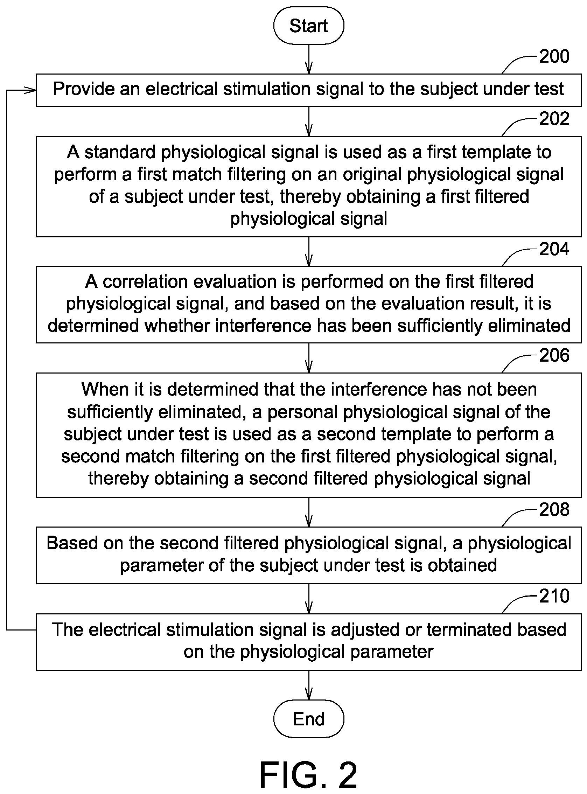

Please refer to FIG. 2, which illustrates a flowchart the physiological signal processing method according to another embodiment of the present disclosure. Compared to the physiological signal processing method shown in FIG. 1, the method in FIG. 2 further includes steps 200 and 210. In step 200, an electrical stimulation signal is provided to the subject under test. In step 210, the electrical stimulation signal is adjusted or terminated based on the physiological parameter. After step 210, for example, the process may return to step 200. Steps 202 to 208 correspond to steps 102 to 108 in FIG. 1 and will not be repeated here.

In one embodiment, the electrical stimulation signal is provided to the subject under test before performing the first match filtering on the original physiological signal of the subject under test. Since the electrical stimulation signal is provided to the subject under test while measuring the physiological signal, the electrical stimulation signal introduces interference into the physiological signal, causing the measured physiological signal to contain significant noise. The embodiments of the present disclosure employ two match filtering operations to efficiently eliminate noise resulting from the interference of the electrical stimulation signal with the physiological signal.

The personal physiological signal of the subject under test may be an initial personal physiological signal measured and obtained from the subject under test before the electrical stimulation signal is provided to the subject under test. Since the initial personal physiological signal is measured and obtained before the electrical stimulation signal is applied to the subject under test, the initial personal physiological signal is not interfered by the electrical stimulation signal and does not contain noise introduced by the electrical stimulation signal. Therefore, the initial personal physiological signal, which is unaffected by noise, is suitable for use as the second template in the second match filtering process to further filter the first filtered physiological signal.

The correlation evaluation performed on the first filtered physiological signal to determine whether interference has been sufficiently eliminated may be achieved by calculating a correlation value between the first filtered physiological signal and the standard physiological signal. When the correlation value is lower than a threshold value, it is determined that the interference has not been sufficiently eliminated. The correlation calculation may be performed by using cross-correlation. By computing the correlation between the first filtered physiological signal and the standard physiological signal, the similarity between the first filtered physiological signal and the standard physiological signal can be determined, thereby estimating the noise level in the first filtered physiological signal. A higher correlation value indicates lower noise levels. That is, the higher the correlation value, the less noise is present. However, the present disclosure is not limited to using correlation calculations to determine the noise level in the first filtered physiological signal, and other methods for assessing the noise level in the first filtered physiological signal may also be applied in embodiments of the present disclosure.

The standard physiological signal may include at least one of a standard electrocardiogram (ECG) signal, a standard photoplethysmography (PPG) signal, and a standard electroencephalogram (EEG) signal. The personal physiological signal may include at least one of a personal electrocardiogram (ECG) signal, a personal photoplethysmography (PPG) signal, and a personal electroencephalogram signal (EEG). These signals have repeatable waveform characteristics, making these signals suitable for use as templates in match filtering. Corresponding match filters can be used to filter the measured physiological signals to eliminate noise.

The physiological parameter may include at least one of a heart rate variability (HRV) parameter, a standard deviation of NN intervals (SDNN) parameter, and a root mean square of successive differences (RMSSD) parameter. These physiological parameters can be used to assess stress levels or the severity of mental health-related disorders and serve as indicators for determining whether the subject under test is abnormal or has recovered to a normal state.

The subject under test may be, for example, a human body (e.g., the body of a user). The electrical stimulation signal may be input via the ear of the subject under test, and the electrical stimulation signal may be an electrical stimulation signal used for treating a disease of the subject under test. The user may wear an ear-mounted device to receive the electrical stimulation signal, such as an electrical current stimulation signal, for treating a disease of the subject under test, for example, mental health-related disorders.

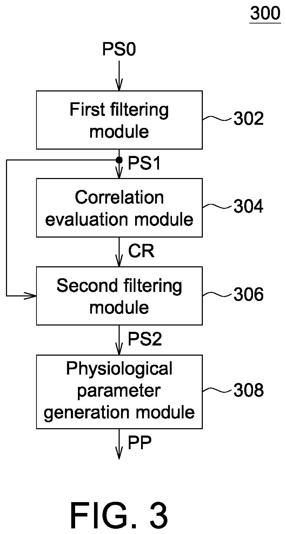

Please refer to FIG. 3, which illustrates a block diagram of a physiological signal processing device according to an embodiment of the present disclosure. The physiological signal processing device 300 includes a first filtering module 302, a correlation evaluation module 304, a second filtering module 306, and a physiological parameter generation module 308. The first filtering module 302 is configured to use a standard physiological signal as a first template to perform a first match filtering on an original physiological signal PS0 of a subject under test, thereby obtaining a first filtered physiological signal PS1. The correlation evaluation module 304 is configured to perform a correlation evaluation on the first filtered physiological signal PS1 and determine, based on the evaluation result, whether interference has been sufficiently eliminated. The second filtering module 306 is configured to, when determining that the interference has not been sufficiently eliminated, use a personal physiological signal of the subject under test as a second template to perform a second match filtering on the first filtered physiological signal PS1, thereby obtaining a second filtered physiological signal PS2. The physiological parameter generation module 308 is configured to generate a physiological parameter PP of the subject under test based on the second filtered physiological signal PS2.

For example, the correlation evaluation module 304 is configured to perform a correlation calculation on the first filtered physiological signal PS1 to obtain a correlation value CR. The second filtering module 306 is configured to, when the correlation value is lower than a threshold value, use a personal physiological signal of the subject under test as a second template to perform a second match filtering on the first filtered physiological signal PS1, thereby obtaining a second filtered physiological signal PS2. The first filtering module 302, the correlation evaluation module 304, the second filtering module 306, and the physiological parameter generation module 308 may be implemented by a processor, a controller, or the like through executing software, firmware, code, or the like, or may be implemented by using related analog or digital circuits. The following example illustrates an implementation where the physiological signal is an electrocardiogram (ECG) signal and the physiological parameter is a heart rate variability (HRV) parameter.

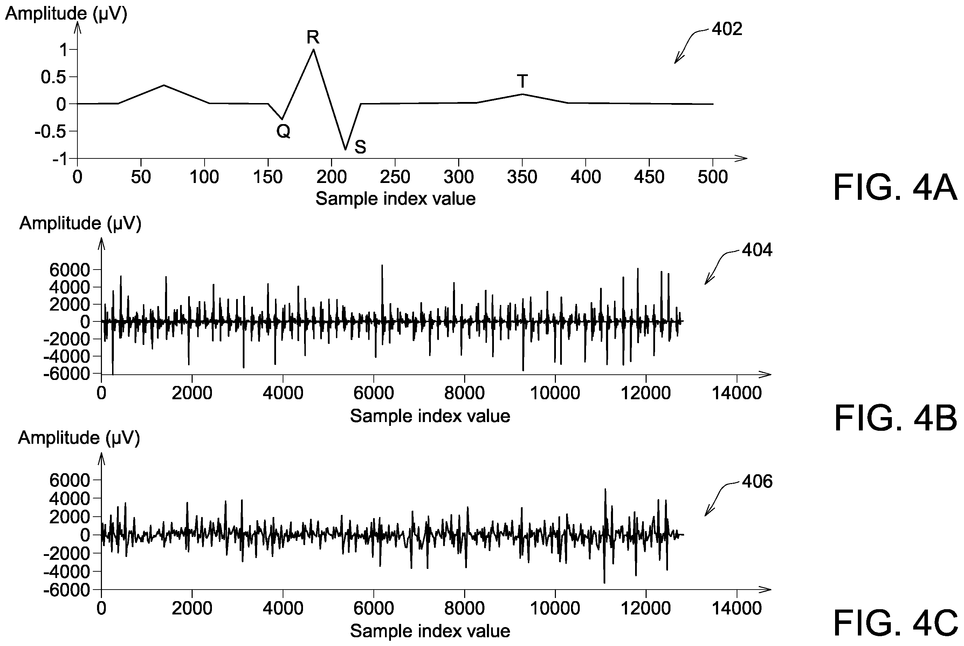

Please refer to FIGS. 4A to 4C. FIG. 4A illustrates an example of a standard electrocardiogram signal. FIG. 4B illustrates an example of an electrocardiogram signal measured from a subject under test when no electrical stimulation signal is provided to the subject under test. FIG. 4C illustrates an example of an electrocardiogram signal obtained by performing match filtering on the measured electrocardiogram signal of FIG. 4B using a match filter with the standard electrocardiogram signal of FIG. 4A as a template.

As shown in FIG. 4A, the standard electrocardiogram signal 402 may be simulated by using several triangular waves of different amplitudes and time periods to represent an actual electrocardiogram signal. For example, triangular waves may be used to represent the Q, R, S, and T waves in the electrocardiogram signal. Based on the standard electrocardiogram signal 402, a match filter using the standard electrocardiogram signal 402 as a template can be designed. The match filter using the standard electrocardiogram signal 402 as a template can remove noise from waveforms that do not conform to the standard electrocardiogram signal 402.

From the measured electrocardiogram signal 404 shown in FIG. 4B, it can be observed that the measured electrocardiogram signal 404 may contain a significant amount of noise, which affects the interpretation of the measured electrocardiogram signal 404. Therefore, by using a match filter with the standard electrocardiogram signal 402 of FIG. 4A as a template to perform match filtering on the measured electrocardiogram signal 404 of FIG. 4B, the noise originally present in the measured electrocardiogram signal 404 of FIG. 4B can be effectively eliminated to obtain the filtered electrocardiogram signal 406 (as shown in FIG. 4C), thereby improving the accuracy of the filtered electrocardiogram signal 406.

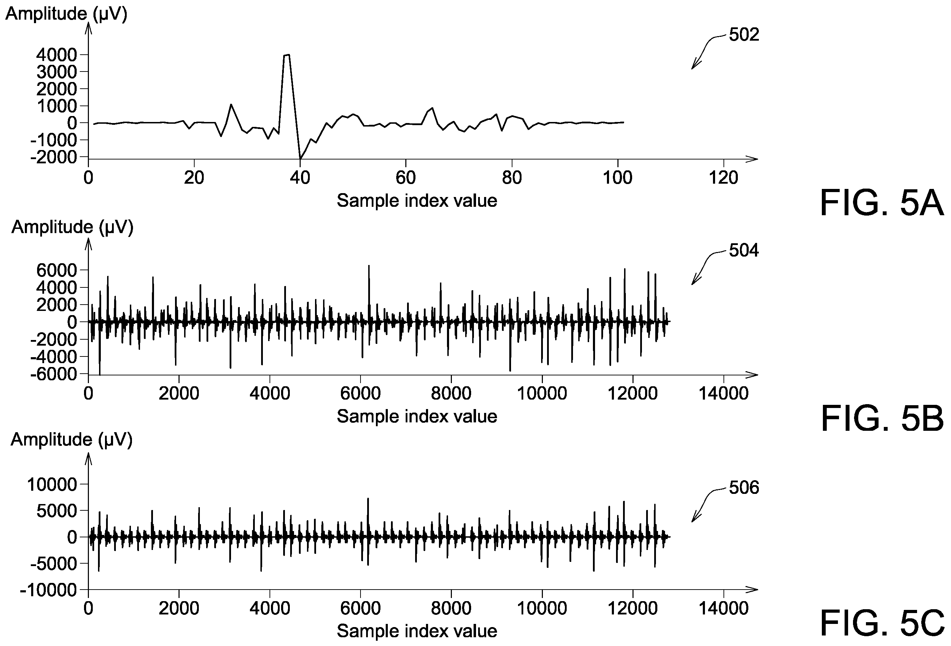

Please refer to FIGS. 5A to 5C. FIG. 5A illustrates an example of a personal electrocardiogram signal. FIG. 5B illustrates an example of an electrocardiogram signal measured from a subject under test when no electrical stimulation signal is provided to the subject under test. FIG. 5C illustrates an example of an electrocardiogram signal obtained by performing match filtering on the measured electrocardiogram signal of FIG. 5B by using a match filter with the personal electrocardiogram signal of FIG. 5A as a template.

As shown in FIG. 5A, the personal electrocardiogram signal 502 may be an electrocardiogram signal actually measured from a subject under test (e.g., a human body) when no electrical stimulation signal is provided to the subject under test. Based on the personal electrocardiogram signal 502, a match filter using the personal electrocardiogram signal 502 as a template can be designed. The match filter using the personal electrocardiogram signal 502 as a template can remove noise that does not conform to the waveform of the personal electrocardiogram signal 502.

As shown in the measured electrocardiogram signal 504 of FIG. 5B, it can be observed that the measured electrocardiogram signal 504 may contain a significant amount of noise, which affects the interpretation of the electrocardiogram signal. However, even after performing match filtering on the measured electrocardiogram signal 504 of FIG. 5B by using a match filter with the personal electrocardiogram signal 502 of FIG. 5A as a template, the filtered electrocardiogram signal 506 obtained (as shown in FIG. 5C) still contains a considerable amount of noise, making it difficult to correctly interpret the filtered electrocardiogram signal 506.

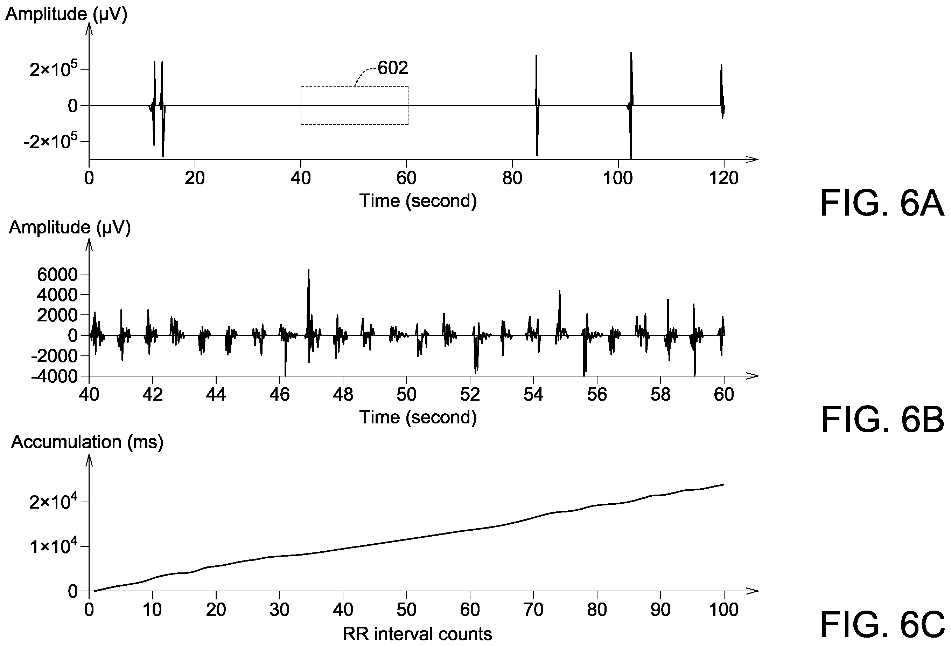

Please refer to FIGS. 6A to 6C. FIG. 6A illustrates a partial waveform of a measured electrocardiogram signal that corresponds to FIG. 4B and was obtained from a subject under test when no electrical stimulation signal was provided, without match filtering. FIG. 6B illustrates part of the magnified measured electrocardiogram signal without match filtering shown in FIG. 6A. FIG. 6C illustrates a schematic diagram of calculating RR interval counts by using the measured electrocardiogram signal without match filtering shown in FIG. 6B.

The part of the magnified measured electrocardiogram signal shown in FIG. 6B corresponds to the part of the magnified measured electrocardiogram signal within the window 602 of FIG. 6A. As shown in FIG. 6B, the measured electrocardiogram signal without match filtering contains a significant amount of noise. Therefore, as illustrated in FIG. 6C, when calculating the number of RR intervals by using the measured electrocardiogram signal of FIG. 6B without match filtering over a time interval of 2 minutes (i.e., 120 seconds), 99 RR intervals are obtained, for example, resulting in a measured heart rate of 99 beats per 2 minutes (i.e., 49.5 beats per minute). Considering that the normal resting heart rate for a healthy individual is typically between 60 and 100 beats per minute, the obtained heart rate of 49.5 beats per minute represents a significant error. The RR interval refers to the time interval between two consecutive R-peaks in the electrocardiogram signal.

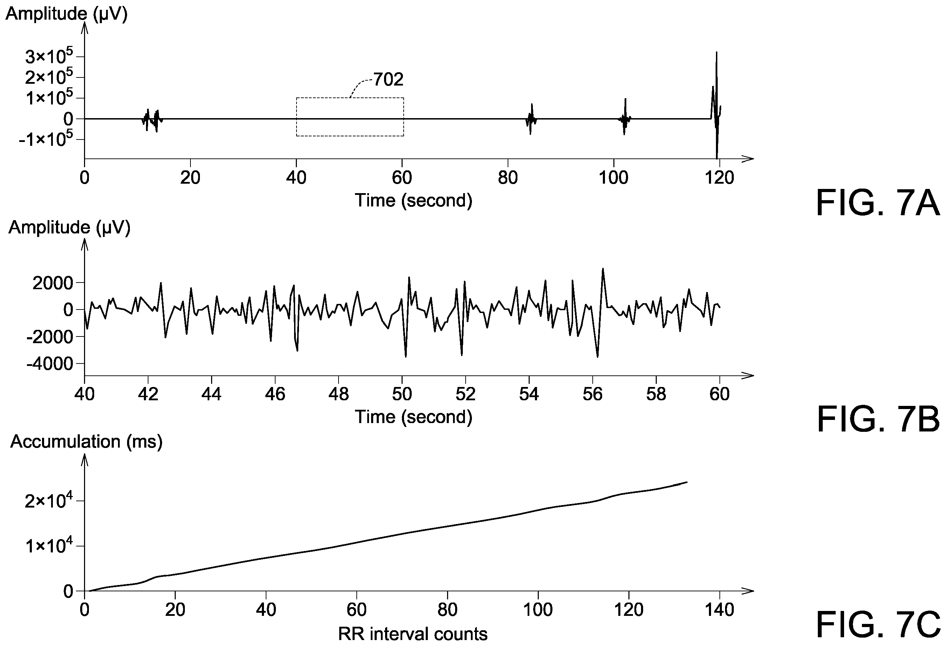

Please refer to FIGS. 7A to 7C. FIG. 7A illustrates a partial waveform of a measured electrocardiogram signal after match filtering when no electrical stimulation signal is provided to the subject under test, corresponding to FIG. 4C. FIG. 7B shows part of the magnified measured electrocardiogram signal in FIG. 7A after match filtering. FIG. 7C illustrates a schematic diagram of RR interval counting by using the measured electrocardiogram signal after match filtering shown in FIG. 7B.

The part of the magnified measured electrocardiogram signal after match filtering in FIG. 7B corresponds to the portion of the measured electrocardiogram signal after match filtering obtained within window 702 of FIG. 7A, where the match filtering was performed by using a standard electrocardiogram signal as a template. As shown in FIG. 7B, the noise in the measured electrocardiogram signal after match filtering has been significantly reduced. Therefore, as shown in FIG. 7C, when calculating the number of RR intervals by using the measured electrocardiogram signal after match filtering of FIG. 7B over a 2-minute (i.e., 120-second) interval, 132 RR intervals are obtained, resulting in a measured heart rate of 132 beats per 2 minutes (i.e., 66 beats per minute). This result aligns more closely with the normal resting heart rate range for a healthy individual, making it a more accurate measurement.

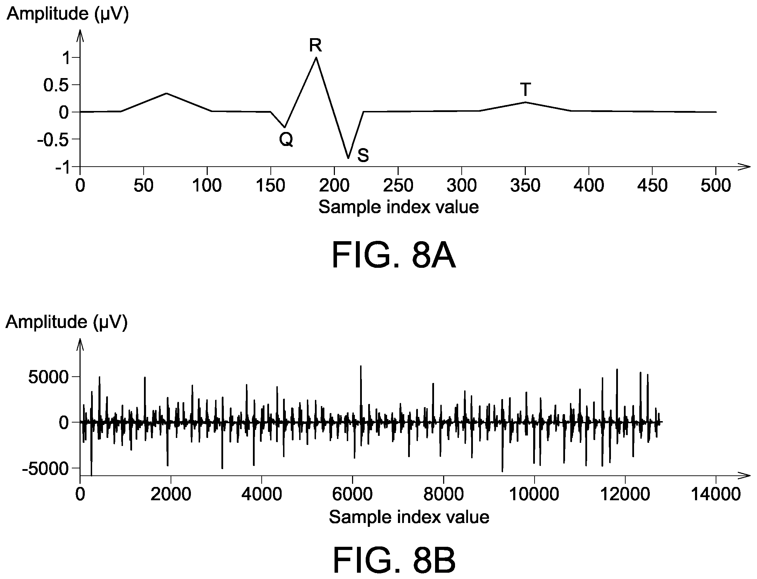

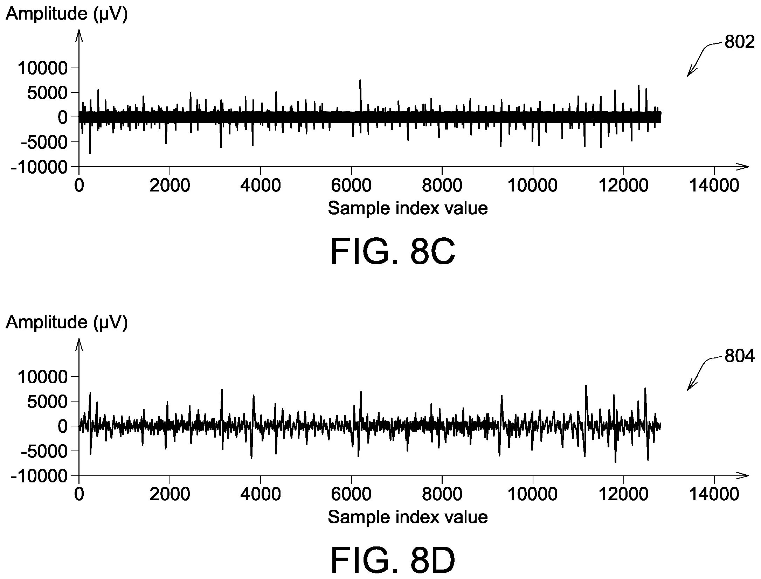

Please refer to FIGS. 8A to 8D. FIG. 8A illustrates an example of a standard electrocardiogram signal (identical to FIG. 4A). FIG. 8B shows an example of a measured electrocardiogram signal when no electrical stimulation signal is provided to the subject under test (identical to FIG. 4B). FIG. 8C illustrates an example of a measured electrocardiogram signal with significant noise when an electrical stimulation signal is applied to the subject under test. FIG. 8D illustrates an example of an electrocardiogram signal obtained by performing match filtering on the electrocardiogram signal with significant noise of FIG. 8C by using a match filter with the standard electrocardiogram signal of FIG. 8A as a template.

As shown in FIG. 8C, the measured electrocardiogram signal 802 contains a significant amount of noise, which affects the interpretation of the measured electrocardiogram signal 802. However, even after performing match filtering on the measured electrocardiogram signal 802 of FIG. 8C by using a match filter with the standard electrocardiogram signal of FIG. 8A as a template, the filtered electrocardiogram signal 804 (as shown in FIG. 8D) still contains a considerable amount of noise, making it difficult to accurately interpret the filtered electrocardiogram signal 804.

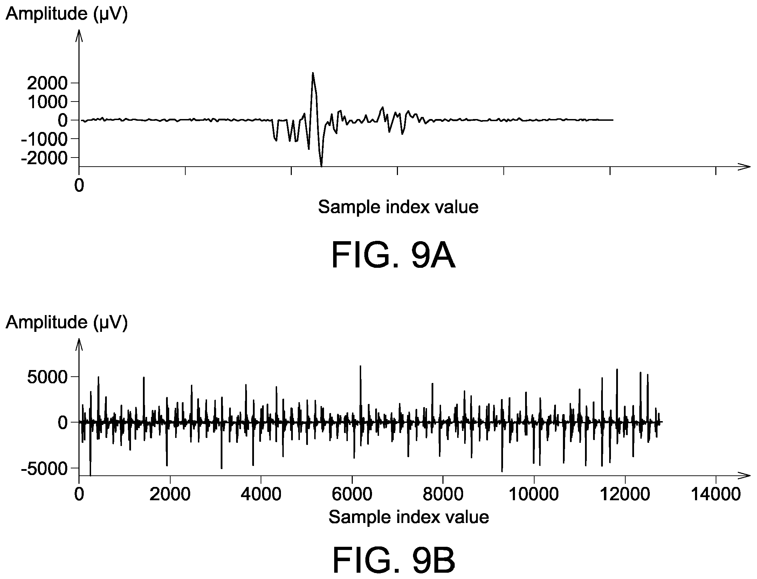

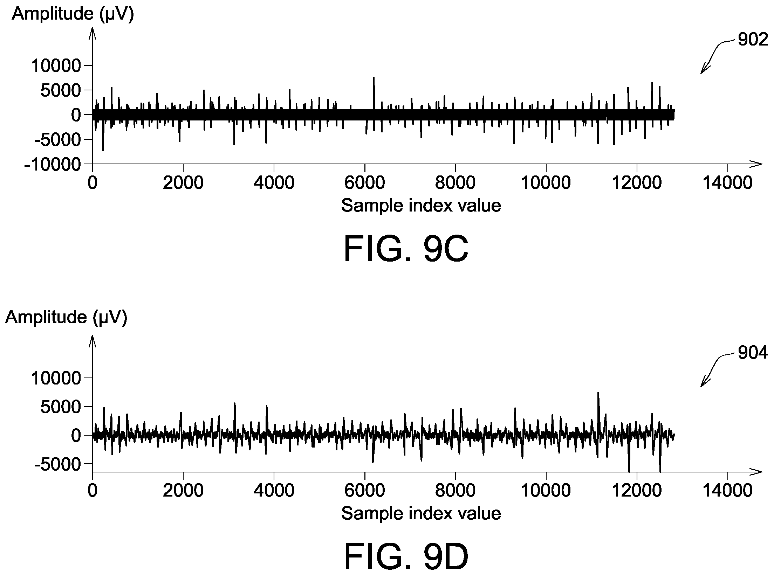

Please refer to FIGS. 9A to 9D. FIG. 9A illustrates an example of a personal electrocardiogram signal. FIG. 9B shows an example of a measured electrocardiogram signal when no electrical stimulation signal is provided to the subject under test (identical to FIG. 5B). FIG. 9C illustrates an example of a measured electrocardiogram signal with significant noise when an electrical stimulation signal is applied to the subject under test. FIG. 9D illustrates an example of an electrocardiogram signal obtained by performing match filtering on the electrocardiogram signal with significant noise of FIG. 9C by using a match filter with the personal electrocardiogram signal of FIG. 9A as a template.

As shown in FIG. 9C, the measured electrocardiogram signal 902 contains a significant amount of noise, which affects the interpretation of the measured electrocardiogram signal 902. After performing match filtering on the measured electrocardiogram signal 902 with significant noise of FIG. 9C by using a match filter with the personal electrocardiogram signal of FIG. 9A as a template, the filtered electrocardiogram signal 904 obtained (as shown in FIG. 9D) has had a large amount of noise removed, allowing the filtered electrocardiogram signal 904 to be accurately interpreted.

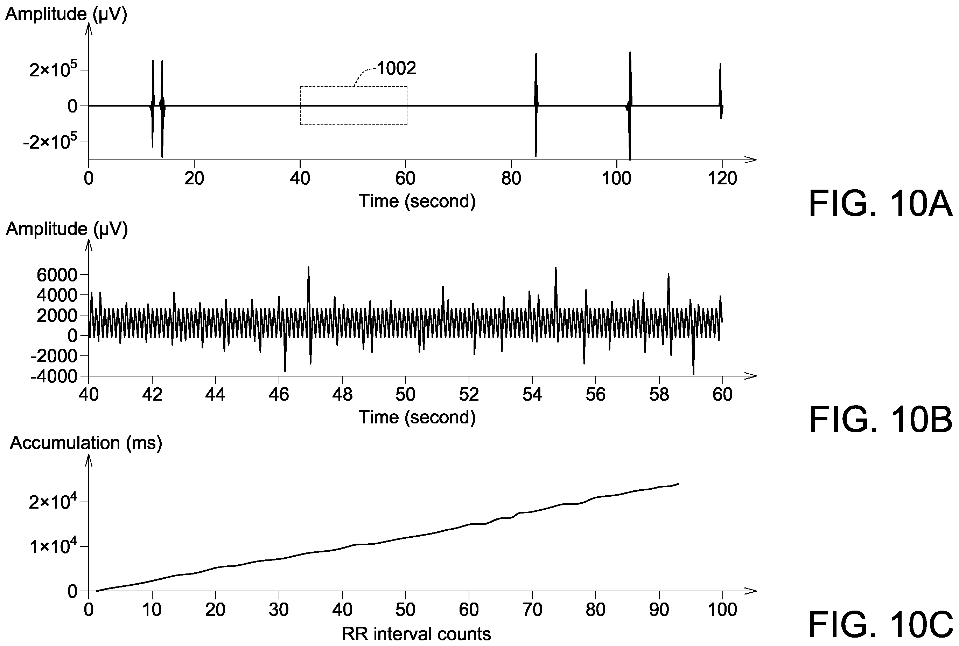

Please refer to FIGS. 10A to 10C. FIG. 10A illustrates a partial waveform of a measured electrocardiogram signal after match filtering which is obtained when an electrical stimulation signal is provided to the subject under test, corresponding to FIG. 8D. FIG. 10B shows part of the magnified measured electrocardiogram signal after match filtering in FIG. 10A. FIG. 10C illustrates a schematic diagram of RR interval counting by using the measured electrocardiogram signal shown in FIG. 10B after match filtering with the standard electrocardiogram signal as a template.

The part of the magnified measured electrocardiogram signal in FIG. 10B corresponds to the portion of the measured electrocardiogram signal obtained within window 1002 of FIG. 10A. As shown in FIG. 10B, the measured electrocardiogram signal after match filtering by using a standard electrocardiogram signal as a template still contains a significant amount of noise. Therefore, as shown in FIG. 10C, when calculating the number of RR intervals by using the measured electrocardiogram signal of FIG. 10B after match filtering over a 2-minute (i.e., 120-second) interval, 92 RR intervals are obtained, resulting in a measured heart rate of 92 beats per 2 minutes (i.e., 46 beats per minute). Considering that the normal resting heart rate for a healthy individual is typically between 60 and 100 beats per minute, the obtained heart rate of 46 beats per minute represents a significant error.

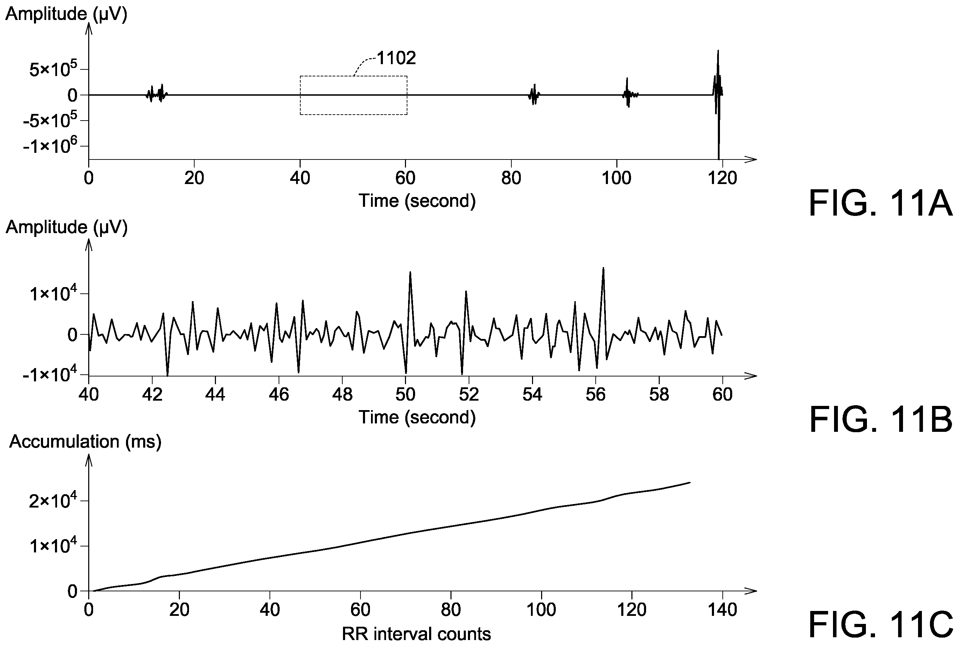

Please refer to FIGS. 11A to 11C. FIG. 11A illustrates a partial waveform of the measured electrocardiogram signal obtained when an electrical stimulation signal is provided to the subject under test after match filtering with the personal electrocardiogram signal as a template, corresponding to FIG. 9D. FIG. 11B shows part of the magnified measured electrocardiogram signal after match filtering in FIG. 11A. FIG. 11C illustrates a schematic diagram of RR interval counting by using the match-filtered measured electrocardiogram signal after match filtering with the personal electrocardiogram signal as a template shown in FIG. 11B.

The part of the magnified match-filtered measured electrocardiogram signal shown in FIG. 11B corresponds to the portion of the match-filtered measured electrocardiogram signal obtained within window 1102 of FIG. 11A, where the match filtering was performed by using a personal electrocardiogram signal as a template. As shown in FIG. 11B, the noise in the match-filtered measured electrocardiogram signal by using a personal electrocardiogram signal as a template has been significantly reduced. Therefore, as shown in FIG. 11C, when calculating the number of RR intervals by using the match-filtered measured electrocardiogram signal with a personal electrocardiogram signal as a template in FIG. 11B over a 2-minute (i.e., 120-second) interval, 130 RR intervals are obtained, resulting in a measured heart rate of 130 beats per 2 minutes (i.e., 65 beats per minute). This result aligns more closely with the normal resting heart rate range for a healthy individual, making it a more accurate measurement.

From the above description, the embodiments of the present disclosure can first perform a first match filtering by using a standard electrocardiogram signal as the first template to remove noise from the electrocardiogram signal. When the electrocardiogram signal after the first match filtering still contains a significant amount of noise, a second match filtering is performed by using a personal electrocardiogram signal as the second template to further remove noise from the electrocardiogram signal. This enables more effective noise removal, improving the accuracy of the electrocardiogram signal. In particular, using the personal electrocardiogram signal as the second template for the second match filtering can more effectively eliminate noise originating from the electrical stimulation signal.

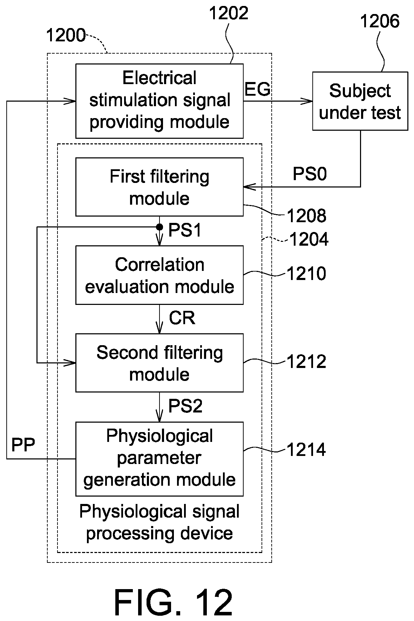

Please refer to FIG. 12, which illustrates a block diagram of an electrical stimulation therapy system 1200 according to an embodiment of the present disclosure. The electrical stimulation therapy system 1200 includes an electrical stimulation signal providing module 1202 and a physiological signal processing device 1204. The electrical stimulation signal providing module 1202 is configured to provide an electrical stimulation signal ES to a subject under test 1206. The physiological signal processing device 1204 includes a first filtering module 1208, a correlation evaluation module 1210, a second filtering module 1212, and a physiological parameter generation module 1214. The first filtering module 1208 is configured to use a standard physiological signal as a first template to perform a first match filtering on an original physiological signal PS0 of the subject under test 1206, thereby obtaining a first filtered physiological signal PS1. The correlation evaluation module 1210 is configured to perform a correlation evaluation on the first filtered physiological signal PS1 and determine, based on the evaluation result, whether interference has been sufficiently eliminated. The second filtering module 1212 is configured to, when determining that the interference has not been sufficiently eliminated, use a personal physiological signal of the subject under test 1206 as a second template to perform a second match filtering on the first filtered physiological signal PS1, thereby obtaining a second filtered physiological signal PS2. The physiological parameter generation module 1214 is configured to generate a physiological parameter PP of the subject under test 1206 based on the second filtered physiological signal PS2. The electrical stimulation signal providing module 1202 is further configured to adjust or terminate the electrical stimulation signal ES based on the physiological parameter PP. The first filtering module 1208, the correlation evaluation module 1210, the second filtering module 1212, and the physiological parameter generation module 1214 may be implemented by a processor, a controller, or the like through executing software, firmware, code, or the like, or may be implemented by using related analog or digital circuits.

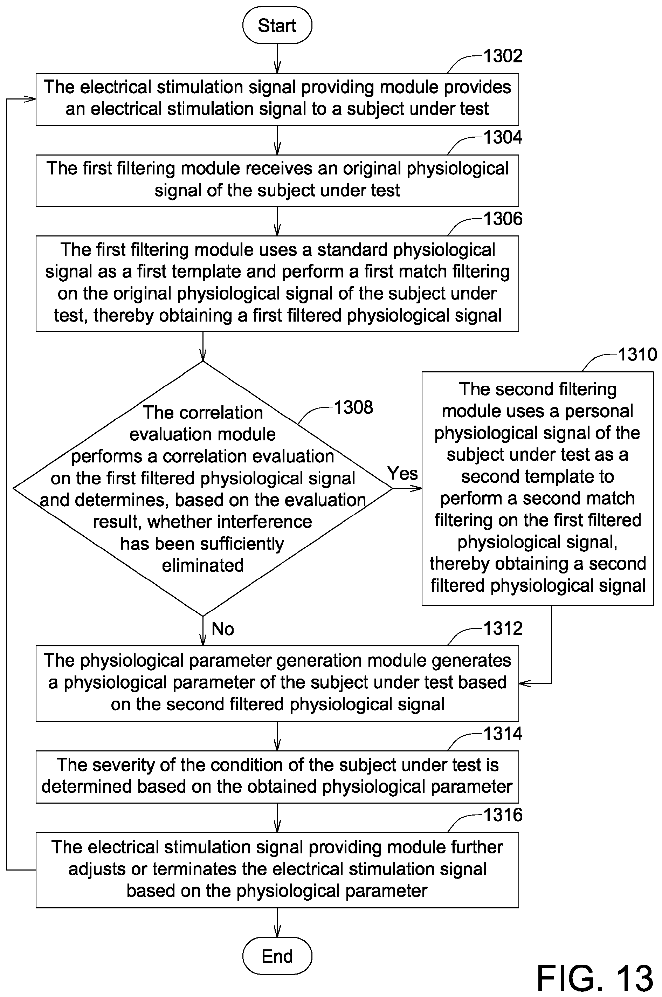

Please refer to FIG. 13, which illustrates a flowchart of an electrical stimulation therapy method 1300 according to an embodiment of the present disclosure. First, in step 1302, the electrical stimulation signal providing module 1202 provides an electrical stimulation signal ES to a subject under test 1206. The electrical stimulation signal ES may be, for example, an electrical current signal that stimulates the vagus nerve of the subject under test 1206. Next, in step 1304, the first filtering module 1208 receives an original physiological signal PS0 of the subject under test 1206, such as an electrocardiogram signal. Then, in step 1306, the first filtering module 1208 uses a standard physiological signal as a first template (e.g., the standard electrocardiogram signal shown in FIG. 4A is used to design a match filter) and perform a first match filtering on the original physiological signal PS0 of the subject under test 1206, thereby obtaining a first filtered physiological signal PS1 (e.g., the filtered electrocardiogram signal shown in FIG. 4C). Some of the noise in the first filtered physiological signal PS1 will be removed.

Next, in step 1308, the correlation evaluation module 1210 performs a correlation evaluation on the first filtered physiological signal PS1 and determines, based on the evaluation result, whether interference has been sufficiently eliminated. The correlation evaluation module 1210 may perform correlation calculations between the first filtered physiological signal PS1 and the standard physiological signal (e.g., the standard electrocardiogram signal shown in FIG. 4A) to obtain a correlation value for evaluating the correlation of the first filtered physiological signal PS1. A higher correlation indicates that lower noise is included in the first filtered physiological signal PS1, meaning a second match filtering is not required. Conversely, a lower correlation indicates that higher noise is included in the first filtered physiological signal PS1, requiring a second match filtering. For example, when the correlation value is lower than a threshold value, the correlation evaluation module 1210 determines that the interference has not been sufficiently eliminated and that a second match filtering is required. In other words, when the correlation value is lower than the threshold value, it indicates lower correlation and higher noise, necessitating a second match filtering.

When it is determined that a second match filtering is required in step 1308, the method proceeds to step 1310; otherwise, the method proceeds to step 1312. In step 1310, the second filtering module 1212 uses a personal physiological signal of the subject under test 1206 as a second template (e.g., uses the personal electrocardiogram signal shown in FIG. 5A as a second template) to perform a second match filtering on the first filtered physiological signal PS1, thereby obtaining a second filtered physiological signal PS2 (e.g., the filtered electrocardiogram signal shown in FIG. 9D). After step 1310, the process proceeds to step 1312.

In step 1312, the physiological parameter generation module 1214 generates a physiological parameter PP of the subject under test 1206 based on the second filtered physiological signal PS2. The physiological parameter PP may be, for example, a heart rate variability parameter. The physiological parameter generation module 1214 may calculate the time intervals between multiple R-wave peaks within a specific time period of the second filtered physiological signal PS2 (e.g., the filtered electrocardiogram signal shown in FIG. 9D) to obtain the heart rate variability parameter.

Next, in step 1314, the severity of the condition of the subject under test 1206 is determined based on the obtained physiological parameter PP. Then, in step 1316, the electrical stimulation signal providing module 1202 further adjusts or terminates the electrical stimulation signal ES based on the physiological parameter PP. For example, the electrical stimulation signal providing module 1202 may determine whether to adjust or terminate the electrical stimulation signal ES based on the severity of the condition of the subject under test 1206 corresponding to the value of the physiological parameter PP.

The standard physiological signal may include at least one of a standard electrocardiogram (ECG) signal, a standard photoplethysmography (PPG) signal, and a standard electroencephalogram (EEG) signal. The personal physiological signal may include at least one of a personal electrocardiogram signal, a personal photoplethysmography signal, and a personal electroencephalogram signal. The physiological parameter may include at least one of a heart rate variability parameter, a standard deviation of NN intervals (SDNN) parameter, or a root mean square of successive differences (RMSSD).

The electrical stimulation signal ES is provided to the subject under test 1206 before performing the first match filtering on the original physiological signal PS0 of the subject under test 1206. When the electrical stimulation signal ES is applied to the subject under test 1206, the physiological signal may be interfered by the noise from the electrical stimulation signal ES.

The personal physiological signal of the subject under test 1206 is an initial personal physiological signal obtained from the subject under test 1206 before the electrical stimulation signal ES is provided to the subject under test 1206. That is, the measurement can be performed on the subject under test 1206 before the electrical stimulation signal ES is applied to the subject under test 1206, while the physiological signal of the subject under test 1206 remains unaffected by noise from the electrical stimulation signal ES. The obtained physiological signal can be used as the personal physiological signal and serve as the template for designing the second match filter to perform the second match filtering.

The subject under test may be, for example, a human body, and the electrical stimulation signal may be input via the ear of the subject under test. The electrical stimulation signal may be an electrical stimulation signal used for treating a disease of the subject under test, such as stress-related mental disorders.

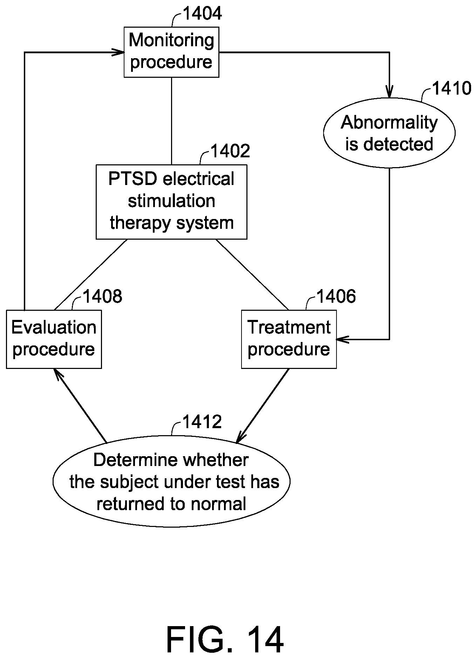

Please refer to FIG. 14, which illustrates an application example of the electrical stimulation therapy system 1200 shown in FIG. 12 and the electrical stimulation therapy method 1300 shown in FIG. 13. Suppose that the electrical stimulation therapy system 1200 of FIG. 12 and the electrical stimulation therapy method 1300 of FIG. 13 are applied in digital therapy for post-traumatic stress disorder (PTSD). As shown in FIG. 14, the digital therapy for PTSD may be controlled by a PTSD electrical stimulation therapy system 1402. PTSD refers to a mental disorder that occurs after experiencing traumatic events such as emotional distress, warfare, traffic accidents, or any severe incidents.

The PTSD electrical stimulation therapy system 1402 may perform a real-time monitoring procedure 1404, a treatment procedure 1406, and an evaluation procedure 1408. The real-time monitoring procedure 1404 may be executed with a wearable device equipped with multiple sensors. The wearable device may be, for example, an ear-worn device. The hardware of the ear-worn device may include an electrical stimulation signal providing module 1202, silicone earphones, a broadband analog front end (AFE) (including a low-noise amplifier (LNA) and an analog-to-digital converter (ADC)). Additionally, the hardware of the ear-worn device may further include a standard photoplethysmography (PPG) sensor, an ExG sensor, or a galvanic skin response (GSR) sensor. The firmware of the ear-worn device may perform gain control, impedance detection and charge balance control, and Bluetooth low energy (BLE) data transmission.

The real-time monitoring procedure 1404 may monitor multiple biomarkers of the subject under test 1206 (e.g., a user wearing the above-mentioned ear-worn device) in real time, for example, by using one or more physiological signals as biomarkers for detection. The physiological signals may include heart rate variability (HRV) signals, photoplethysmography (PPG) signals, electroencephalogram (EEG) signals, and galvanic skin response (GSR) signals.

The PTSD electrical stimulation therapy system 1402 may convert the above-mentioned biomarker information into a neuroactivity index and integrate relevant physiological data into an Al model for training to determine whether abnormalities have occurred. When an abnormality is detected, as shown in block 1410, the PTSD electrical stimulation therapy system 1402 executes the treatment procedure 1406. For example, the PTSD electrical stimulation therapy system 1402 activates the electrical stimulation signal providing module 1202, which delivers an electrical signal (e.g., an electrical current signal) to the subject under test 1206. The electrical signal stimulates the vagus nerve of the subject under test 1206 as a method of treatment.

The occurrence of an abnormal condition may be determined when the parasympathetic nervous system activity index falls below a predetermined threshold, indicating a PTSD episode. The parasympathetic nervous system activity index may be determined based on abnormalities in the standard deviation of NN intervals (SDNN) parameter or the root mean square of successive differences (RMSSD) parameter, or by evaluating whether the ratio of low-frequency power (LF) to high-frequency power (HF) in the heart rate variability signal falls within a predetermined range. After executing the treatment procedure 1406, the PTSD electrical stimulation therapy system 1402 may determine whether the subject under test 1206 has returned to normal by determining whether the parasympathetic nervous system activity index has recovered, as shown in block 1412. Based on this evaluation, it may be decided whether to adjust or terminate the electrical stimulation signal ES.

The PTSD electrical stimulation therapy system 1402 may also track the progress of the treatment by analyzing objective physiological signal feedback or subjective self-assessments completed by the subject under test 1206. The system may then evaluate whether to adjust or terminate the electrical stimulation signal ES. For example, the intensity of the electrical stimulation signal ES may be increased or decreased, or the electrical stimulation signal ES may be stopped entirely. The objective physiological signal feedback may include heart rate variability signals (HRV), photoplethysmography (PPG) signals, electroencephalogram (EEG) signals, and galvanic skin response (GSR) signals, which are fed back into the system for evaluation. Additionally, physiological signal feedback may be obtained from heart rate variability (HRV) parameters, standard deviation of NN intervals (SDNN) parameters, and root mean square of successive differences (RMSSD) parameters generated from electrocardiogram signals.

Furthermore, the frequency-domain characteristics of the heart rate variability (HRV) signal may serve as an indicator for determining whether the subject under test 1206 has returned to normal. For example, the low-frequency (LF) power of the heart rate variability (HRV) signal (e.g., the signal power within the frequency range of 0.04-0.15 Hz) and the high-frequency (HF) power of the heart rate variability (HRV) signal (e.g., the signal power within the frequency range of 0.15-0.4 Hz) may be analyzed as biomarkers. The clinical significance of LF power is that it represents both sympathetic and parasympathetic nervous system activity, while HF power represents parasympathetic nervous system activity.

In clinical medicine, stress typically increases sympathetic nervous system regulation of the heart while reducing parasympathetic nervous system regulation. When an individual experiences increased stress, the LF power of the heart rate variability (HRV) signal, which represents both sympathetic and parasympathetic nervous activity, increases, while the HF power, which represents parasympathetic nervous activity, decreases. As a result, the ratio of LF to HF, which represents the balance of autonomic nervous system activity, also increases. Therefore, by calculating the LF-to-HF ratio (LF/HF), it is possible to determine whether the parasympathetic nervous system activity index has fallen below a predetermined threshold and thereby detect abnormalities.

Additionally, heart rate variability (HRV) signals are commonly used to study the relationship between anxiety disorders and depression and cardiovascular diseases. Regarding anxiety disorders, a consistent finding is that the high-frequency power (HF) of heart rate variability (HRV) signals decreases, indicating a reduction in parasympathetic nervous system regulation and a loss of autonomic nervous system flexibility. Therefore, abnormalities in the subject under test 1206 may also be determined by assessing whether the HF power falls below a threshold value.

Furthermore, according to clinical trials of digital therapy for stress-related mental disorders, heart rate variability (HRV) signals exhibit specific patterns in physiological mental disorders such as generalized anxiety disorder (GAD), major depressive disorder (MDD), panic disorder, and somatic symptom disorder (SSD). The heart rate variability (HRV) parameter values for these four disorders are lower than those of healthy individuals, with the most significant decrease observed in major depressive disorder. Generalized anxiety disorder shows SDNN and RMSSD second only to those of major depressive disorder patients. Therefore, by analyzing and comparing heart rate variability (HRV) parameters, SDNN parameters, and RMSSD parameters, it is possible to determine whether the subject under test 1206 has any of these conditions and whether they have returned to normal after treatment.

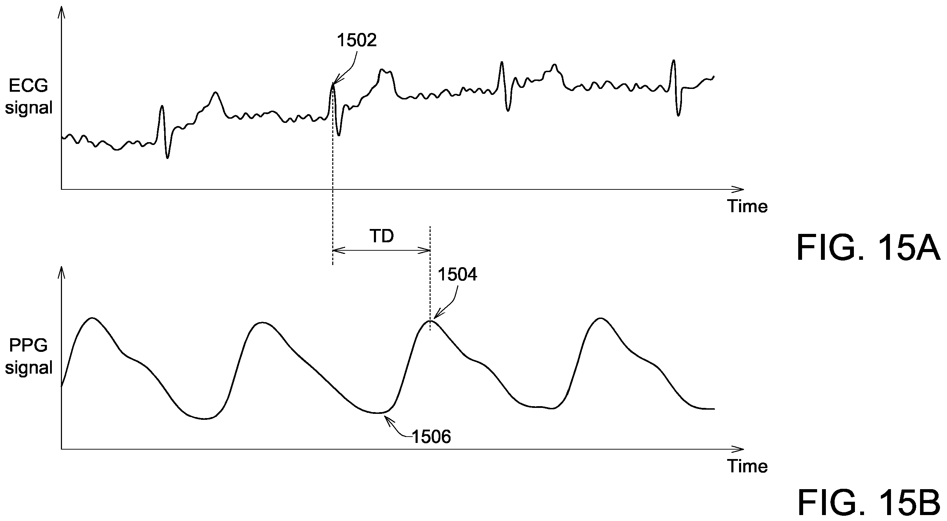

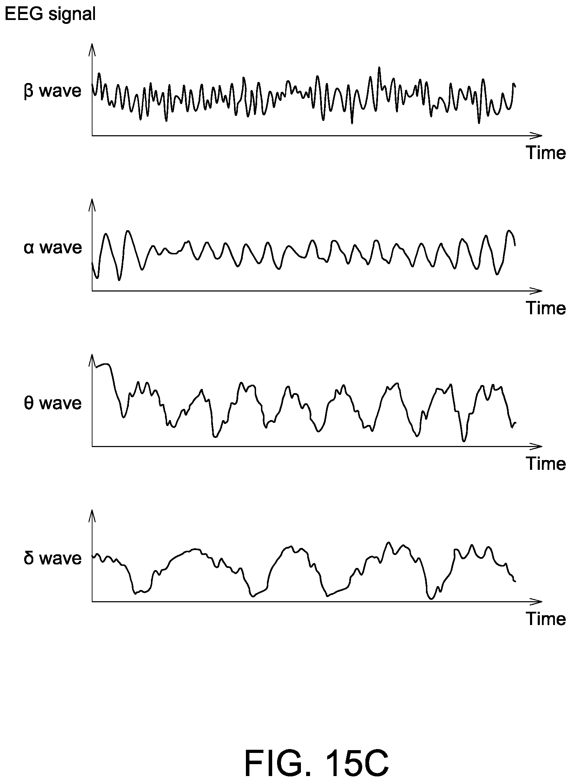

Please refer to FIGS. 15A to 15C. FIG. 15A illustrates an example of an electrocardiogram (ECG) signal as a physiological signal according to an embodiment of the present disclosure. FIG. 15B illustrates an example of a standard photoplethysmography (PPG) signal as a physiological signal according to an embodiment of the present disclosure. FIG. 15C illustrates an example of an electroencephalogram (EEG) signal as a physiological signal according to an embodiment of the present disclosure. A time delay TD exists between the R-wave peak 1502 in FIG. 15A and the pulse peak 1504 in FIG. 15B. The pulse peak 1504 in FIG. 15B corresponds to the heart's contraction, while the pulse valley 1506 in FIG. 15B corresponds to the heart's relaxation. The electroencephalogram signal in FIG. 15C contains β waves, α waves, θ waves, and δ waves, which correspond to brain wave activity. The physiological signals shown in FIGS. 15A to 15C, including electrocardiogram (ECG) signals, photoplethysmography (PPG) signals, and electroencephalogram (EEG) signals, have fixed waveform patterns that occur repetitively. These signals can be used as templates in match filtering to design match filters for noise removal.

The match filter may be designed using the following method. The purpose of the match filter is to maximize the signal-to-noise ratio (SNR) of the target signal, allowing for the detection and extraction of specific patterns or features in a signal. Suppose a target signal s(t) and an observed signal x(t) exist, and the goal is to extract the target signal s(t) from the observed signal x(t).

The match filter is defined as follows. Suppose the target signal s(t) and the observed signal x(t) are continuous-time signals. In the case of discrete-time signals, the target signal can be represented as s[n], and the observed signal can be represented as x[n]. The basic form of the match filter involves convolving the observed signal x[n] with the reverse signal of the target signal s[n] (i.e. time-reversed version of the target signal s[n]), resulting in the output y[n]. That is, y[n] = x[n] * s[-n], where "*" represents the convolution operation, and s[-n] is the time-reversed version of the target signal s[n]. Applying this to the present disclosure, the standard physiological signal is used as the first template, meaning that the standard physiological signal serves as the target signal s1[n]. Simultaneously, the original physiological signal of the subject under test is used as the observed signal x1[n], which is convolved with the time-reversed standard physiological signal s1[-n] to perform the first match filtering. The resulting output y1[n] is the first filtered physiological signal obtained through the first match filtering, where y1[n] = x1[n] * s1[-n].

Furthermore, when using the personal physiological signal as the second template, the personal physiological signal serves as the target signal s2[n]. The first filtered physiological signal is used as the observed signal x2[n], which is then convolved with the time-reversed personal physiological signal s2[-n] to perform the second match filtering. The resulting output y2[n] is the second filtered physiological signal obtained through the second match filtering, where y2[n] = x2[n] * s2[-n].

The physiological signal processing method and device of the present disclosure, along with the electrical stimulation therapy system utilizing them, effectively address interference issues in physiological signals caused by electrical stimulation. For example, when electrical current is used to stimulate the vagus nerve in the ear, the induced interference in electrocardiogram (ECG) signals may affect signal accuracy. When electrical current stimulation is applied to the vagus nerve in the ear, the received electrocardiogram (ECG) signal may be disrupted, resulting in errors that compromise electrocardiogram signal accuracy. By employing the match filtering technique, interference signals can be effectively removed, ensuring the accuracy and stability of physiological signals.

Additionally, the physiological signal processing method and device of the present disclosure, as well as the electrical stimulation therapy system that uses them, can generate physiological parameters from physiological signals to assess the severity of mental disorders and determine whether to continue electrical stimulation, thus achieving closed-loop control. Since physiological signal accuracy is critical for the application of this technology, the noise suppression filtering technique of the present disclosure is suitable not only for clinical medical environments but also for various applications requiring high-precision physiological signal monitoring. It provides reliable technical support for subsequent physiological signal detection and management. Therefore, the embodiments of the present disclosure enhance the accuracy of physiological signal detection, achieve noise suppression, and offer dynamic adaptability and diagnostic accuracy. Additionally, the physiological signal processing method and device of the present disclosure, as well as the electrical stimulation therapy system reduce error rates, adapt to various application scenarios, and improve overall signal processing performance.

Since external noise interference may affect physiological signal characteristics under different conditions, the embodiments of the present disclosure employ dual match filtering to enable more flexible noise suppression by leveraging the different characteristics of two templates. Specifically, in the context of electrical stimulation, physiological signals may be distorted due to the electrical stimulation, and dual match filtering can better capture and enhance effective physiological signals.

It will be apparent to those skilled in the art that various modifications and variations can be made to the disclosed embodiments. It is intended that the specification and examples be considered as exemplars only, with a true scope of the disclosure being indicated by the following claims and their equivalents.

## Claims

1. A method for processing a physiological signal, comprising:
using a standard physiological signal as a first template to perform a first match filtering on an original physiological signal of a subject under test (1206), thereby obtaining a first filtered physiological signal;
performing a correlation evaluation on the first filtered physiological signal and determining, based on an evaluation result, whether interference has been sufficiently eliminated;
when determining that the interference has not been sufficiently eliminated, using a personal physiological signal of the subject under test (1206) as a second template to perform a second match filtering on the first filtered physiological signal, thereby obtaining a second filtered physiological signal; and
generating a physiological parameter of the subject under test (1206) based on the second filtered physiological signal.

2. The method for processing a physiological signal according to claim 1, further comprising:
providing an electrical stimulation signal to the subject under test (1206); and
adjusting or terminating the electrical stimulation signal based on the physiological parameter.

3. The method for processing a physiological signal according to claim 2, wherein the electrical stimulation signal is provided to the subject under test (1206) before performing the first match filtering on the original physiological signal of the subject under test (1206);
wherein the personal physiological signal of the subject under test (1206) is an initial personal physiological signal measured and obtained from the subject under test (1206) before the electrical stimulation signal is provided to the subject under test (1206).

4. The method for processing a physiological signal according to anyone of the claims 1 to 3, wherein performing the correlation evaluation on the first filtered physiological signal and determining, based on an evaluation result, whether the interference has been sufficiently eliminated is achieved by calculating a correlation value between the first filtered physiological signal and the standard physiological signal, wherein when the correlation value is less than a threshold value, it is determined that the interference has not been sufficiently eliminated;
wherein the standard physiological signal comprises at least one of a standard electrocardiogram (ECG) signal, a standard photoplethysmography (PPG) signal, and a standard electroencephalogram (EEG) signal, the personal physiological signal comprises at least one of a personal electrocardiogram signal, a personal photoplethysmography signal, and a personal electroencephalogram signal, and the physiological parameter comprises at least one of a heart rate variability (HRV) parameter, a standard deviation of NN intervals (SDNN) parameter, and a root mean square of successive differences (RMSSD) parameter.

5. The method for processing a physiological signal according to anyone of the claims 1 to 4, wherein the subject under test is a human body, the electrical stimulation signal is input via an ear of the subject under test, and the electrical stimulation signal is an electrical stimulation signal for treating a disease of the subject under test.

6. A physiological signal processing device (300, 1204), comprising:
a first filtering module (302, 1208), configured to use a standard physiological signal as a first template to perform a first match filtering on an original physiological signal of a subject under test (1206), thereby obtaining a first filtered physiological signal;
a correlation evaluation module (304, 1210), configured to perform a correlation evaluation on the first filtered physiological signal and determine, based on an evaluation result, whether interference has been sufficiently eliminated;
a second filtering module (306, 1212), configured to, when determining that the interference has not been sufficiently eliminated, use a personal physiological signal of the subject under test (1206) as a second template to perform a second match filtering on the first filtered physiological signal, thereby obtaining a second filtered physiological signal; and
a physiological parameter generation module (308, 1214), configured to generate a physiological parameter of the subject under test (1206) based on the second filtered physiological signal.

7. The physiological signal processing device (300, 1204) according to claim 6, wherein an electrical stimulation signal is provided to the subject under test (1206), and the electrical stimulation signal is adjusted or terminated based on the physiological parameter.

8. The physiological signal processing device (300, 1204) according to claim 7, wherein the electrical stimulation signal is provided to the subject under test (1206) before performing the first match filtering on the original physiological signal of the subject under test (1206);
wherein the personal physiological signal of the subject under test (1206) is an initial personal physiological signal measured and obtained from the subject under test (1206) before the electrical stimulation signal is provided to the subject under test (1206).

9. The physiological signal processing device (300, 1204) according to anyone of the claims 6 to 8, wherein the correlation evaluation module (304, 1210) performs the correlation evaluation of the first filtered physiological signal by calculating a correlation value between the first filtered physiological signal and the standard physiological signal, wherein when the correlation value is less than a threshold value, it is determined that the interference has not been sufficiently eliminated;
wherein the standard physiological signal comprises at least one of a standard electrocardiogram (ECG) signal, a standard photoplethysmography (PPG) signal, and a standard electroencephalogram (EEG) signal, the personal physiological signal comprises at least one of a personal electrocardiogram (ECG) signal, a personal photoplethysmography (PPG) signal, and a personal electroencephalogram (EEG) signal, and the physiological parameter comprises at least one of a heart rate variability (HRV) parameter, a standard deviation of NN intervals (SDNN) parameter, and a root mean square of successive differences (RMSSD) parameter.

10. The physiological signal processing device according to anyone of the claims 6 to 9, wherein the subject under test is a human body, the electrical stimulation signal is input via an ear of the subject under test, and the electrical stimulation signal is an electrical stimulation signal for treating a disease of the subject under test.

11. An electrical stimulation therapy system (1200), comprising:
an electrical stimulation signal providing module (1202), configured to provide an electrical stimulation signal to a subject under test (1206); and
a physiological signal processing device (300, 1204), comprising:
a first filtering module (302, 1208), configured to use a standard physiological signal as a first template to perform a first match filtering on an original physiological signal of the subject under test (1206), thereby obtaining a first filtered physiological signal;
a correlation evaluation module (304, 1210), configured to perform a correlation evaluation on the first filtered physiological signal and determine, based on an evaluation result, whether interference has been sufficiently eliminated;
a second filtering module (306, 1212), configured to, when determining that the interference has not been sufficiently eliminated, use a personal physiological signal of the subject under test (1206) as a second template to perform a second match filtering on the first filtered physiological signal, thereby obtaining a second filtered physiological signal; and
a physiological parameter generation module (308, 1214), configured to generate a physiological parameter of the subject under test (1206) based on the second filtered physiological signal;
wherein the electrical stimulation signal providing module (1202) is further configured to adjust or terminate the electrical stimulation signal based on the physiological parameter.

12. The electrical stimulation therapy system (1200) according to claim 11, wherein the electrical stimulation signal is provided to the subject under test (1206) before performing the first match filtering on the original physiological signal of the subject under test (1206);
wherein the personal physiological signal of the subject under test (1206) is an initial personal physiological signal measured and obtained from the subject under test (1206) before the electrical stimulation signal is provided to the subject under test (1206).

13. The electrical stimulation therapy system (1200) according to claim 11 or 12, wherein the correlation evaluation module (304, 1210) performs the correlation evaluation of the first filtered physiological signal by calculating a correlation value between the first filtered physiological signal and the standard physiological signal, and when the correlation value is less than a threshold value, it is determined that the interference has not been sufficiently eliminated.

14. The electrical stimulation therapy system (1200) according to anyone of the claims 11 to 13, wherein the standard physiological signal comprises at least one of a standard electrocardiogram (ECG) signal, a standard photoplethysmography (PPG) signal, and a standard electroencephalogram (EEG) signal, the personal physiological signal comprises at least one of a personal electrocardiogram signal, a personal photoplethysmography signal, and a personal electroencephalogram signal, the physiological parameter comprises at least one of a heart rate variability (HRV) parameter, a standard deviation of NN intervals (SDNN) parameter, and a root mean square of successive differences (RMSSD) parameter.

15. The electrical stimulation therapy system (1200) according to anyone of the claims 11 to 14, wherein the subject under test (1206) is a human body, the electrical stimulation signal is input via an ear of the subject under test (1206), and the electrical stimulation signal is an electrical stimulation signal for treating a disease of the subject under test (1206).
